Europäisches Patentamt

European Patent Office

Office européen des brevets

(19)

(11) Publication number: **0 377 044 B1**

## EUROPEAN PATENT SPECIFICATION

(12)

(45) Date of publication of patent specification: **04.08.93**  (51) Int. Cl.5: **A61K 37/24**

(21) Application number: **89904210.5**

(22) Date of filing: **04.04.89**

(86) International application number:
**PCT/JP89/00359**

(87) International publication number:
**WO 89/09613 (19.10.89 89/25)**

(54) **PROTECTIVE AGENT FOR RADIATION HAZARD.**

(30) Priority: **04.04.88 JP 81210/88**

(43) Date of publication of application:
**11.07.90 Bulletin 90/28**

(45) Publication of the grant of the patent:
**04.08.93 Bulletin 93/31**

(84) Designated Contracting States:
**BE CH DE FR GB IT LI NL SE**

(56) References cited:
**JP-A- 5 852 225**
**JP-A-54 148 722**

**INT. J. CANCER, vol. 28, 1981, pages 59-64; E. LEGRAND et al.: "Influence of serum thymic factor (FTS) on radiation-induced leukaemogenesis in thymectomized AKR mice"**

(73) Proprietor: **MITSUI TOATSU CHEMICALS, Inc.**
**2-5 Kasumigaseki 3-chome**
**Chiyoda-Ku Tokyo 100(JP)**

(72) Inventor: **SHIKITA, Mikio**
**13-1, Kohinata 2-chome Bunkyo-ku**
**Tokyo 112(JP)**
Inventor: **AWAYA, Akira Dainiapato**
**1541, Yabecho Totsuka-ku**
**Yokohama-shi Kanagawa 244(JP)**
Inventor: **KOBAYASHI, Hisashi**
**640-46, Miyanodaiapato 2141, Togo**
**Mobara-shi Chiba 297(JP)**
Inventor: **ISHIZUKA, Yusaku**
**21, Honmokuosatocho Naka-ku**
**Yokohama-shi Kanagawa 231(JP)**
Inventor: **ABE, Hayao**
**640-16, Miyanodaiapato 2141, Togo**
**Mobara-shi Chiba 297(JP)**

(74) Representative: **Vossius & Partner**
**Siebertstrasse 4 P.O. Box 86 07 67**
**W-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 377 044 B1

## Description

The present invention relates to the preparation of a medicament for use in humans or animals. More particularly the present invention relates to the preparation of a medicament having protective activity against different' radiation damages or a medicament capable of promoting the recovery of radiation damages such as hematopoietic dysfunctions.

Recently damages caused by electromagnetic waves for medical use such as X-rays or $\gamma$-rays or radiations such as cosmic rays or even ultraviolet rays as contained in the sunlight, in particular those radiation damages in the radiotherapy for malignant tumors, cancers and leukemia, have been at issue. Radiotherapy, side by side with surgical treatment is a radical treatment for the remedy of cancers. Appreciation and expectations of its utility, however, have been attenuating due to early damages which develop in patients because of exposure to radiation. Such early damages include hematopoietic dysfunctions, local damages such as fibrosis in organs, skins or mucosas, and gastrointestinal damages. The alleviation and recovery of hematopoietic dysfunctions, in particular, are essential to the improvement of patients in prognosis and life span extension.

Development of a protective agent for the prevention and remedy of such damages caused by radiation will make it possible to drastically increase doses with which to irradiate affected parts in the radiotherapy and thus contribute to further enhancement of radiotherapeutic effects on cancers.

How to protect patients from early effects of radiation such as dermal erythema or pigmentation, late effects of radiation such as radiation burns on skins, hematopoietic dysfunctions, aging acceleration or life span shortening, and in particular the danger of secondary carcinogenesis due to the radiotherapy, is a problem of importance to be solved.

In addition, increase of patients with cancers is a matter of concern due to genetic effects, carcinogenic activity and the like of exposure to radiation of radiation handlers or reactor operating personnel as well as of exposure to more intense ultraviolet rays resulting from the destruction of the ozonosphere in the upper stratosphere due to fluorocarbon gases or the like.

The human skin is sensitive to ultraviolet rays in the wavelength range of 280 to 320 nm, and those in the range of 305 to 310 nm are believed to cause skin cancers. It has been discussed that the destruction of the ozonosphere which absorbs the ultraviolet rays in this wavelength region would increase the ultraviolet rays pouring down onto the earth and thus lead to the increase, as mentioned above, of skin cancers. It is becoming an important task to develop a medicament for the prevention and remedy of such damages due to radiation exposure.

As agents for protecting the living body from radiation exposure, sulfur-containing amine compounds have been studied, but not yet clinically applied. Recently in the United States of America, Amifostine (Walter Reed Army Inst.) is under clinical investigation as a radio-protective agent but not without problems in respect of adverse side effects.

[Disclosure of the Invention]

With the aim of developing a medicament which unlike chemotherapeutics such as Amifostine potentiates the defensive ability inherent in the living body, the present inventors have made extensive screening for substances, useful as a protective agent for the prevention and remedy of radiation damages, in respect of a variety of high safety peptides of natural origin.

As a result, it has now been found that a nonapeptide known as facteur thymique serique, hereinafter referred to as FTS, and its derivatives or salts ensure the inhibition of decease, and therefore survival or life span extension, of X-radiated mice. The present invention has been accomplished on the basis of the above finding and provides a protective agent for the prevention and remedy of different damages caused by X-rays, $\gamma$-rays, ultraviolet rays or the like.

The present inventors previously found that FTS was suitable as a therapeutic agent for multiple sclerosis, Guillain-Barre syndrome, inflammatory neuritis, polyneuritis and other various diseases accompanying immunodeficiency such as immunological demyelinating diseases, and provided such therapeutic agents (Japanese Laid-open Patent Appln. No. Sho. 58-52225). The fact that a nonapeptide known as FTS exhibits preventive and therapeutic effects in the protection from radiation damages is quite unexpected from the prior art and was discovered for the first time by the present inventors.

In accordance with the present invention, there is provided a radioprotective agent, characterized by containing as an effective ingredient thereof a nonapeptide having the following amino acid sequence:

pGlu-Ala-Lys-Ser-Gln-Gly-Gly-Ser-Asn

or an ester or amide at the carboxyl group of the C-terminal of the asparagine or a pharmacologically

2

acceptable salt thereof.

The nonapeptide used in the present invention can be prepared without difficulty according to a liquid phase or solid phase peptide synthetic method conventionally employed for the synthesis of usual peptides (Concerning these methods, please refer to Japanese Laid-open Patent Appln. No. Sho. 54-16425 and U.S.P. 4,301,065). Alternatively, the nonapeptide can be prepared also by a genotechnological or cell technological procedure.

The esters at the carboxyl group of C-terminal of the asparagine in the nonapeptide used in the present invention are those of the carboxylic acid pharmacologically acceptable and examples of such esters include methyl ester, ethyl ester, propyl ester, isopropyl ester, n-butyl ester, isobutyl ester, tert-butyl ester, n-pentyl ester, isopentyl ester, neopentyl ester, tert-pentyl ester, n-hexyl ester, sec-hexyl ester, heptyl ester, octyl ester, sec-octyl ester, tert-octyl ester, nonyl ester, decyl ester, undecyl ester, dodecyl ester, tridecyl ester, tetradecyl ester, hexadecyl ester, octadecyl ester, nonadecyl ester, eicosyl ester, cyclopentyl ester, cyclohexyl ester, cycloheptyl ester, cyclooctyl ester, allyl ester, isopropenyl ester, benzyl ester, o-, m- or p-chlorobenzyl ester, o-, m- or p-fluorobenzyl ester, o-, m- or p-bromobenzyl ester, o-, m-or p-iodobenzyl ester, o-, m- or p-methylbenzyl ester, o-, m- or p-ethylbenzyl ester, o-, m- or p-isopropylbenzyl ester, cinnamyl ester, aminoethyl ester, o-, m- or p-aminobenzyl ester, o-, m- or p-nitrobenzyl ester, o-, m- or p-methoxybenzyl ester, o-, m- or p-ethoxybenzyl ester, o-, m- or p-aminophenethyl ester, $\alpha$-furfuryl ester, $\alpha$-thienylmethyl ester, $\alpha$-pyridylmethyl ester, $\alpha$-pyridylethyl ester, piperidinomethyl ester, $\alpha$-piperidylmethyl ester, morpholinomethyl ester, $\alpha$-morpholinylmethyl ester. The amides at the carboxyl group of C-terminal of the asparagine in the nonapeptide used in the present invention are those of carboxylic acid pharmacologically acceptable and examples of such amides include amide itself, methylamide, ethylamide, propylamide, isopropylamide, n-butylamide, isobutylamide, tert-butylamide, n-pentylamide, isopentylamide, neopentylamide, tert-pentylamide, n-hexylamide, sec-hexylamide, heptylamide, octylamide, sec-octylamide, tert-octylamide, nonylamide, decylamide, undecylamide, dodecylamide, tridecylamide, tetradecylamide, hexadecylamide, octadecylamide, nonadecylamide, eicosylamide, cyclopentylamide, cyclohexylamide, cycloheptylamide, cyclooctylamide, allylamide, isopropenyl-amide, benzylamide, o-, m- or p-chlorobenzylamide, o-, m- or p-fluorobenzylamide, o-, m- or p-bromobenzylamide, o-, m- or p-iodobenzylamide, o-, m- or p-methylbenzylamide, o-, m- or p-ethylbenzylamide, o-, m- or p-isopropylbenzylamide, cinnamylamide, aminoethylamide, o-, m- or p-aminobenzylamide, o-, m- or p-nitrobenzylamide, o-, m- or p-methoxybenzylamide, o-, m- or p-ethoxybenzylamide, o-, m- or p-aminophenethylamlde, $\alpha$-furfurylamide, $\alpha$-thienylmethylamide, $\alpha$-pyridylmethylamide, $\alpha$-pyridylethylamide, piperidinomethylamide, $\alpha$-piperidylmethylamide, morpholinoethylamide, $\alpha$-morpholinylmethylamide, methoxycarbonyl-($\alpha$-mercaptomethyl)-methylamide and ethoxycarbonyl- ($\alpha$-mercaptomethyl)methylamide.

The above mentioned pharmacologically acceptable salts include acid-additions salts at the amino group of the nonapeptide and salts with bases at the carboxylic acid of the nonapeptide. The acid-addition salts include those with organic acids and inorganic acids. Illustrative of these salts are, for example, salts with carboxylic acids such as formic acid, acetic acid, propionic acid, trifluoroacetic acid, tartaric acid, fumaric acid, malic acid, maleic acid, oxalic acid and naphthoic acid and salts with sulfonic acids such as methanesulfonic acid, p-toluenesulfonic acid and naphthalenesulfonic acid as well as salts with inorganic acids such as hydrochloric acid, sulfuric acid, nitric acid and phosphoric acid.

The above mentioned salts with bases include salts with inorganic bases, such as alkali metal salts, alkaline earth metal salts and ammonium salts as well as salts with organic bases, i.e. salts with amines. Illustrative of these salts are lithium salt, sodium salt, potassium salt, calcium salt, ammonium salt, triethyl amine salt, ethanolamine salt, tris salt and dicyclohexylamine salt.

The radioprotective agent of the present invention can be prepared in a usual conventional pharmaceutical manner according to the type of dosage form. An effective ingredient selected from the nonapeptide, an ester, an amide and a salt thereof is processed properly with a pharmacologically acceptable carrier, excipient or diluent to a suitable type of dosage form. The dosage form can be made in any of the various types suitable for the route of administration such as external application, oral administration, non-oral administration, etc.

The effect of the radioprotective agent of the present invention is confirmed by the experiments as described below. Thus, mice, rats, guinea pigs or the like mammals were subjected to whole body irradiation, using a X-radiation unit (Shimazu Seisaku-sho, Shin-Ai 250 II), to determine % survival over a period of thirty days. In carrying out these experiments, given amounts of the nonapeptide were administered by different routes of administration, such as intraperitoneal, intravenous, intramuscular, subcutaneous or oral route of administration, either every day or every other day over a given period of time from before or from immediately after the irradiation, to measure the body-weight of the animals and observe whether they were alive or dead. According to the results of these experiments, for example, all the mice in a control group died, whereas the % survival for a group of the animals to which the medicament of the present invention had been administered was observed to be 40 - 100% depending on the dose of radiation. In addition, it was observed that also in respect of deaths the medicament-administered group showed a significant or marked life span-extending effect over the control group. Also in respect of body weight, the medicament-administered group exhibited a significant inhibition of body-weight loss as compared with the control group.

On the other hand, animals were subjected to whole body irradiation with lethal or sub-lethal doses and changes with the lapse of time in the number of blood cells were measured for given periods of time in order to check the medicament for the promotive effect on the recovery of hematopoietic dysfunctions. Blood cells used as index included red blood cells, reticulocytes, white blood cells such as lymphocytes and neutrophils, platelets, hemoglobins, hematocrit and the like. According to the administration of the medicament of the present invention, the reduction of these blood cells due to irradiation was observed to be markedly inhibited, and the recovery to a normal range to be accelerated. In addition, immune responses of immunocytes, i.e. the competence of spleen cells to respond to Concanavalin A (Con A) and the reactivity of thymus cells to Interleukin-1 (IL-1), were examined every day for the medicament-administered group until all animals deceased, to find that they were apparently higher than those for the control group. Also in respect of immunocytes capabilities of hunoral factor production, i.e. the IL-1 producing capability of peritoneal macrophage (Mø) and the capability of spleen cells and peritoneal Mø to produce colony stimulating factor (CSF), the medicament-administered group was found to be superior to the control group. Thus, as is evident from these observations, the medicament of the present invention is believed to act on hematopoietic organs, e.g. bone marrow and spleen, and exert the life span-extending or life-saving effect due to its inhibitory or recovery-promoting effect on hematopoietic dysfunctions, and it is therefore valuable as a preventive and therapeutic agent against radiation damages.

Furthermore, in radio therapeutic experiments on cancer-carrying mice, by way of pre- or post-irradiation administration of the medicament according to the invention, the anti-cancer effect was observed to be potentiated and side effects caused by irradiation were also alleviated, it following that doses usable for the therapy of cancers can effectively be augmented. Moreover, in spite of the fact that irradiation of mice is known to cause cancer, the administration over a certain period of the medicament according to the present invention inhibits the development of radiation-induced cancers in mice.

To check any toxicity of the effective ingredient of the medicament of this invention, 100 mg/kg of the effective ingredient was subcutaneously administered every day for consecutive 14 days to mice whereupon no abnormal symptom was found by external check. Further, 30 mg/kg of the effective ingredient was subcutaneously administered every day to rats for consecutive 21 days whereupon no abnormal symptom was found in external behavior observation and biochemical diagnosis of sera and in a result of pathological anatomy. Thus, the medicament of the present invention is a safe medicament of extremely low toxicity and can be administered for a long period of time.

As animals to which the medicament of this invention can be administered, there can be mentioned, for example, human, domestic animals such as cattle, horse, pig, sheep, goat, rabbit, dog and cat, mammalia kept in zoo or the like such as lion, elephant, giraffe, bear, gorilla, monkey and chimpanzee, various test animals such as mouse, rat, guinea pig and the like, domestic fowls such as fowl, and pets such as birds, reptiles, amphibia and fishes. The dose of the medicament is usually 0.1 μg - 500 mg/day for 1 kg body weight cf these animals. The medicament in these doses may be administered, for example, in 1-6 portions in a day. The dose may properly be increased or decreased according to ages, symptoms, etc. of the objects to be administered. No limitation exists in the route of administration of the medicament, but it may be administered by intravenous, intramuscular, intracutaneous or subcutaneous injection. The medicament can be processed to an ointment which can be applied to eyes, oral cavity, nasal cavity, skin or the like. The medicament can be administered in the form of a suppository, a jelly, eye drops, nasal drops, preparations absorbable in nasal or oral cavity, an aerosol, a spray, oral preparations or the like. In order to prevent the effective ingredient from rapid decomposition or inactivation in the living body, the effective ingredient may be processed with appropriate pharmaceutical ingredients, for example, alcoholic, oily or fatty physiologically harmless solid or liquid materials such as lecithin or a suspension liposome thereof to medical preparations in which the activity is maintained for a long period of time.

The medicament of the present invention can be administered with other medicines, for example, biological response modifiers such as immunopotentiators and glutathione, adenine cr cepharantin preparations known to have leukopenia-recovering activity, or alternatively may be incorporated with these as a combination agent to enhance clinical effects.

The present invention will now be illustrated in more detail by way of examples and experimental examples, but the present invention is not limited by these examples.

Example 1 A vial preparation for injection

In a distilled water was dissolved 1 mg of FTS•CH$_3$COOH•2H$_2$O (prepared by Mitsui Seiyaku Kogyo KK) and the solution was subjected to sterilizing filtration, charged into a vial and then subjected to lyophilization.

Example 2 An ampoule preparation for injection

In physiological saline was dissolved 5 mg of FTS•CH$_3$COOH•2H$_2$O (prepared by Mitsui Seiyaku Kogyo KK) and the solution was subjected to sterilizing filtration and charged into an ampoule.

Example 3 An injection preparation for subcutaneous injection

In a 2% carboxymethylcellulose PBS (physiological saline buffered with a phosphate) was suspended 2 mg per unit dose of FTS•CH$_3$COOH•2H$_2$O (prepared by Mitsui Seiyaku Kogyo KK). The suspension was mixed with Lipomal comprising soybean phosphatide (prepared by Huhtamaki OY/Leiras Pharmaceuticals Co.) or Intralipid (prepared by Cutter Laboratories) as an oil-in-water type emulsion for intravenous injection. In case of using Lipomal, the PBS solution dissolving FTS was mixed with an equiamount of Lipomal. In case of using Intralipid, 2.5 ml of the PBS solution dissolving FTS was mixed with 0.1 ml of Tween® 80 (prepared by Sigma Chemicals Inc.) and 4.6 ml of Intralipid.

Example 4 A liposome preparation

There are 3 kinds of liposome preparations which are different in electric charge from one another. The liposome preparations are classified by their structures into 4 kinds.
There are 3 kinds of electric charge: neutral, positive and negative. In view of the structure, there are known 4 kinds; a multilaminar liposome (MLV, multilamellar vesicle), a small unilaminar liposome (SUV, small unilamellar vesicle), a large unilaminar liposome (LUV, large unilamellar vesicle) and one having a structure similar to LUV but having several lamellae (REV, reverse-phase evaporation vesicle).

(1) A neutral electric charge liposome enclosing FTS:

A phospholipid such as phosphatidylcholine or sphingomyelin and a solution of cholesterol in chloroform were mixed in a mole ratio of 2:1, 4:1 or 1:1 and the solvent was once removed from the mixture by distillation under reduced pressure. A solution of 1/100 - 1/1000 equivalent of FTS in PBS (physiological saline buffered with a phosphate) was added to the mixture and the whole was well mixed by the aid of a Vortex mixer whereby MLV was obtained.
This was then subjected to an ultrasonic treatment above a phase transition temperature (Tc) of the phospholipid whereby SUV was obtained.
An aqueous solution of calcium chloride was added to the resultant SUV and the mixture was incubated for 1 hour at 37°C to effect hybridization. EDTA was then added and the mixture was incubated for 30 minutes at 37°C to eliminate Ca$^{++}$ whereby LUV was obtained.
The method for preparing REV is as follows: After removing the solvent from a chloroform solution of the lipid by distillation under reduced pressure, a proper amount of diethyl ether is added to dissolve the lipid satisfactorily. A PBS solution of FTS is added to the solution and the mixture is subjected to an ultrasonic treatment to obtain a homogeneous uniphase solution. After concentrating the resultant solution under reduced pressure at room temperature, the PBS solution is added to the residue and the mixture was mixed well by the aid of a Vortex mixer to obtain REV.

(2) A positively charged liposome enclosing FTS:

Except that the constituents of the lipid are different, the method for preparing the liposome is same as that in case of the above mentioned neutral electric charge liposome.
A phospholipid such as phosphatidylcholine or sphingomyelin, cholesterol, and a positively charged higher aliphatic amine such as stearylamine were mixed together in a molar ratio of 7:2:1 or 4:1:1 to form a lipid ingredient, and FTS was enclosed in a similar manner.

(3) A negatively charged liposome enclosing FTS:

A phospholipid such as phosphatidylcholine or sphingomyelin, cholesterol, and a negatively charged higher aliphatic ester such as dicetyl phosphate or sulfatide were mixed together in a molar ratio of 7:2:1 or 4:1:1 to form a lipid ingredient, and FTS was enclosed in a similar manner.

Example 5 An ointment

In purified water was dissolved 2 mg of FTS•$CH_3COOH$•$2H_2O$ (prepared by Mitsui Seiyaku Kogyo KK). Next, 25 g of white vaseline, 20 g of stearyl alcohol, 4 g of HCO-60 and 1 g of glycerol monostearate were weighed and mixed. A previously prepared aqueous solution (containing FTS) of 12 g of propylene glycol, 0.1 g of methyl p-hydroxybenzonate and 0.1 g of propyl p-hydroxybenzonate were added to the mixture and the whole was thoroughly blended to form an emulsion which was then mixed until it was cooled and solidified.

Example 6 A suppository

In a hard fat previously warmed was dispersed 10 mg of FTS•$CH_3COOH$•$2H_2O$ (prepared by Mitsui Seiyaku Kogyo KK). The total amount was adjusted to 2 g.

Example 7 A capsule for nasal use

In 29.95 mg of miglyol 812 neutral oil (Dynamite Nobel Co.) was dissolved under a sterilizing condition 0.05 mg of FTS. This solution was charged into a conventional unit-dose applicator, which was mounted on a driving capsule just before the use.

Example 8 Nasal drops

In distilled water were dissolved at room temperature sodium dihydrogen phosphate, disodium hydrogen phosphate, sodium chloride and EDTA-disodium salt in amounts shown below. FTS was dissolved in this solution which was then filtered through a membrane filter.

| | |
|---|---|
| FTS | 0.10 mg |
| Sodium monohydrogen phosphate $2H_2O$ | 0.30 mg |
| Sodium dihydrogen phosphate $12H_2O$ | 10.10 mg |
| Benzalconium chloride | 0.10 mg |
| Ethylenediamine tetraacetic acid disodium salt (EDTA) | 0.50 mg |
| Sodium chloride | 4.50 mg |
| Distilled water | 987.60 mg |
| pH value | 5.0 ± 0.3 |

Example 9 A nasal spray preparation

In hydroxypropylcellulose or hydroxypropylmethylcellulose was suspended 2 mg of FTS•$CH_3COOH$•$2H_2O$ (prepared by Mitsui Seiyaku Kogyo KK). The suspension was processed to a spray preparation by the aid of a spray-preparing machine.

Given below are examples for pharmacological experiments and toxicity experiments concerning the medicament of this invention.

Experimental Example 1 Effects on mice irradiated with 800 roentgen (R)

Male, 10 week-old C3H/He mice were used and groups of 10 - 15 animals each were prepared.

The mice were subjected to whole body irradiation for 8 minutes using a X-radiation unit (manufactured by Shimazu Seisaku-sho, Shin-Ai 250 II). These animals received, once per day, subcutaneous administration of 100 μg of FTS•$CH_3COOH$•$2H_2O$ as used in Example 1 for consecutive 14 days. For a pre-administered group, the administration started two days before the irradiation. For a post-administered

6

EP 0 377 044 B1

group, the first administration was effected immediately after the irradiation. As control groups, one group which received only once subcutaneous administration of 1 KE of Picibanil immediately after the irradiation and another group which received every day administration of physiological saline were prepared. The results are shown in Table 1. As is apparent from Table 1, it was revealed that the medicament of the invention clearly prevents or inhibits the dying of mice due to irradiation, thus being valuable as an agent for the prevention and remedy of radiation damages. In particular, the post-administered group showed more marked such effects.

7

Table 1 Effects on mice irradiated with 800R X-rays

| Administration | The number of animals used | The number of days and survivals after X-radiation | | | | | | | | | | | | |
|---|---|---|---|---|---|---|---|---|---|---|---|---|---|---|
| | | 10 | 11 | 12 | 13 | 14 | 15 | 16 | 17 | 18 | 19 | 20 | 25 | 30 (days) |
| Physiological saline | 10 | 10 | 10 | 9 | 8 | 8 | 3 | 1 | 0 | | | | | |
| Pre-administration of medicament (100µg) | 15 | 15 | 15 | 15 | 15 | 15 | 14 | 13 | 11 | 11 | 11 | 9 | 9 | 9 |
| Post-administration of medicament (100µg) | 15 | 15 | 15 | 15 | 14 | 13 | 10 | 8 | 6 | 6 | 6 | 6 | 5 | 5 |
| Post-administration of Picibanil (1 KE) | 10 | 10 | 10 | 9 | 7 | 7 | 5 | 1 | 0 | | | | | |

Experimental Example 2 Effects on mice irradiated with 900R

In the same manner as in Experimental Example 1, mice were subcutaneously administered, after X-radiation for 9 minutes, with 100 µg/day of FTS•CH₃COOH•2H₂O, once a day and twelve times in all, i.e.

on the zero, 1st, 2nd, 3rd, 4th, 5th, 7th, 8th, 9th, 10th, 11th and 12th days. The results are shown in Table 2. The FTS-administered group exhibited a life span extension against the exposure to radiation at a dose of as high as 900R.

Table 2  Effects on mice irradiated with 900R X-rays

| Administration | The number of animals used | The number of days and survivals after X-radiation | | | | | | | | | | | |
| --- | --- | --- | --- | --- | --- | --- | --- | --- | --- | --- | --- | --- | --- |
| | | 9 | 10 | 11 | 12 | 13 | 14 | 15 | 16 | 18 | 20 | 21 | 23 (days) |
| Physiological saline | 15 | 15 | 13 | 13 | 4 | 3 | 0 | | | | | | |
| Post-administration of medicament | 15 | 15 | 13 | 12 | 12 | 7 | 4 | 3 | 2 | 2 | 2 | 1 | 0 |

Experimental Example 3 Effects on mice irradiated with 600R

In the same manner as in Experimental Examples 1 and 2, mice were irradiated with X-rays for 6 minutes. For a period of consecutive 14 days from 2 days before irradiation, the animals were subcutaneously administered, once a day, with 100 $\mu$g/day of FTS•CH$_3$COOH•2H$_2$O as medicament.

Although the X-radiation was at a sublethal dose, four out of ten animals of the control group survived, i.e. more than half died, in 19 days, whereas all ten out of ten animals of the medicament-administered group survived. The results are shown in Table 3.

10

Table 3   Effects on mice irradiated with 600R X-rays

| Administration | The number of animals used | The number of days and survivals after X-radiation | | | | | | | | | | | | |
| | | 8 | 9 | 10 | 11 | 12 | 13 | 14 | 15 | 16 | 17 | 18 | 19 | 30 (days) |
|---|---|---|---|---|---|---|---|---|---|---|---|---|---|---|
| Administration of Physiological saline | 10 | 10 | 10 | 10 | 9 | 8 | 8 | 7 | 7 | 6 | 5 | 5 | 4 | 4 |
| Pre-administration of medicament (100μg) | 10 | 10 | 10 | 10 | 10 | 10 | 10 | 10 | 10 | 10 | 10 | 10 | 10 | 10 |

Experimental Example 4 Blood test

Independently of the irradiation carried out in Experimental Example 3 with mice for life/death observation, mice were subjected to 600R X-radiation to conduct a blood test on a dayly basis. Another

11

group of mice was prepared and subjected to 400R X-radiation. Each of the 600R- and 400R-irradiated groups was further divided into two, one `group being for pre-administration with 100 $\mu$g of the above mentioned medicament and the other group serving as a control. Samples of blood were taken every day from each mouse and tested for the number white blood cells (WBC), that of red blood cells (RBC), that of reticulocytes (RET), that of blood platelet (PLT), that of hemoglobin (HGB) and hematocrit value (HCT).

A representative example is shown in Table 4. It was shown that all these blood parameters were apparently improved by the administration of the medicament as compared with those for the control group.

Table 4   Blood test

| Treatment | The number of animals | Blood parameters | | | | | |
| --- | --- | --- | --- | --- | --- | --- | --- |
| | | WBC | RBC | RET | PLT | HGB | HCT |
| Normal group | 3 | 52.6±9.4 | 749.3±7.5 | 26.0±6.6 | 556.3±28.8 | 13.3±0.1 | 41.2±0.3 |
| 600 X-rays, physiological saline, on the 7th day | 5 | 3.8±1.6 | 547.0±26.8 | 13.2±5.4 | 162.8±5.3 | 9.6±0.3 | 30.3±1.0 |
| 600R X-rays, medicament, on the 7th day | 5 | 34.2±25.2* | 691.8±96.1* | 24.6±10.9 | 372.0±174.7* | 12.3±1.9* | 39.1±5.5** |
| 400R X-rays, physiological saline, on the 14th day | 3 | 3.3±0.9 | 397.0±160.2 | 14.0±4.5 | 76.6±35.4 | 6.0±2.1 | 22.0±8.8 |
| 400R X-rays, medicament, on the 14th day | 3 | 8.7±0.4*** | 522.7±21.7 | 30.0±6.5* | 240.6±14.3** | 8.8±0.3 | 29.8±1.4 |

t-test: *p<0.05, **p<0.01, ***p<0.001

Experimental Example 5 Body weight measurement

The body weight of each experimental group of mice in Experimental Example 3 was measured on consecutive days for observation of changes in weight. The results are shown in Table 5. It was shown that the medicament-administered group evidently weighed more than the control group.

13

Table 5    Body weight

body weight (g)    [Mean ± S.D.]
(The number of animals)

| The number of days after irradiation | 1 | 3 | 5 | 7 | 9 | 11 |
|---|---|---|---|---|---|---|
| 600R X-ray, physiological saline | 27.4±1.0 (15) | 26.5±0.9 (15) | 26.2±0.8 (15) | 26.2±0.9 (10) | 26.8±0.6 (10) | 25.2±0.9 (9) |
| 600R X-ray, medicament | 27.8±1.5 (15) | 27.7±1.1** (15) | 27.9±1.1*** (15) | 28.8±1.2*** (10) | 29.8±1.0*** (10) | 30.2±0.3*** (10) |

| The number of days after irradiation | 13 | 15 | 17 | 19 | 29 |
|---|---|---|---|---|---|
| 600R X-ray, physiological saline | 25.1±0.6 (8) | 25.6±0.9 (7) | 26.4±1.0 (5) | 27.0±0.7 (4) | 24.3±1.5 (4) |
| 600R X-ray, medicament | 29.6±0.7*** (10) | 28.3±0.8*** (10) | 28.2±1.0** (10) | 29.1±0.8*** (10) | 28.5±1.4*** (10) |

t-test: ***$p<0.001$, **$p<0.01$

Experimental Example 6 Toxicity test

No toxicity was observed when 50 mg/kg and 100 mg/kg of effective ingredient were subcutaneously administered for consecutive 14 days to a group of five ddy male mice of 5 weeks old.

Experimental Example 7 Toxicity test

No toxicity was observed when 30 mg/kg of effective ingredient was subcutaneously administered for consecutive 21 days to a group of ten Wister rats of 5 weeks old.

As mentioned in the foregoing, few effective medicaments other than leukopenia-recovering agents, which are used only to a small extent, have been available to date in the medical field of radiation damage protection. Thus, the medicament of the present invention is epoch-making in that it brings about excellent preventive/therapeutic effects on radiation damages through activation of the immune or biological defense system. In addition, since it is a peptide of animal origin and a naturally occurring substance, the nonapeptide (FTS) used in the present invention is, unlike FTS analogues with similar amino acid sequences, of no toxicity in the living body, and presents no problems like antigenicity or anaphylactic shock. The medicament of the invention can therefore be used as safe and useful medicines in humans and animals.

**Claims**

1. Use of a nonapeptide having the following amino acid sequence:
   PGlu-Ala-Lys-Ser-Gln-Gly-Gly-Ser-Asn
   or an ester or amide at the carboxyl group of the c-terminal of the asparagine or a pharmacologically acceptable salt thereof for the preparation of a radioprotective agent.

**Patentansprüche**

1. Verwendung eines Nonapeptids der folgenden Aminosäuresequenz:
   pGlu-Ala-Lys-Ser-Gln-Gly-Gly-Ser-Asn
   oder eines Esters oder eines Amids an der Carboxylgruppe des C-Terminus des Asparagins oder eines pharmazeutisch verträglichen Salzes davon für die Herstellung eines Strahlenschutzmittels.

**Revendications**

1. Utilisation d'un nonapeptide ayant la séquence suivante des amino-acides :
   pGlu-Ala-Lys-Ser-Gln-Gly-Gly-Ser-Asn
   ou d'un ester ou amide du groupe carboxyle de l'extrémité C-terminale de l'asparagine ou d'un sel pharmacologiquement acceptable de celui-ci pour la préparation d'un agent radioprotecteur.